(19) **Europäisches Patentamt / European Patent Office / Office européen des brevets**

(11) **EP 4 009 995 B1**

(12) # EUROPEAN PATENT SPECIFICATION

(45) Date of publication and mention of the grant of the patent:
**16.10.2024 Bulletin 2024/42**

(21) Application number: **20765075.5**

(22) Date of filing: **06.08.2020**

(51) International Patent Classification (IPC):
**A61K 35/744** $^{(2015.01)}$  **A61K 35/745** $^{(2015.01)}$
**A61K 35/747** $^{(2015.01)}$  **A61K 35/742** $^{(2015.01)}$
**A61K 35/741** $^{(2015.01)}$  **A61P 1/00** $^{(2006.01)}$
**A23L 33/135** $^{(2016.01)}$

(52) Cooperative Patent Classification (CPC):
(C-Sets available)
**A61K 35/744; A23L 33/135; A61K 35/741; A61K 35/742; A61K 35/745; A61K 35/747; A61P 1/00** (Cont.)

(86) International application number:
**PCT/IB2020/057426**

(87) International publication number:
**WO 2021/024216 (11.02.2021 Gazette 2021/06)**

(54) **PROBIOTIC MIXTURE**

PROBIOTISCHE MISCHUNG

MÉLANGE PROBIOTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **08.08.2019 IT 201900014388**

(43) Date of publication of application:
**15.06.2022 Bulletin 2022/24**

(73) Proprietor: **Università Cattolica del Sacro Cuore 20123 Milano MI (IT)**

(72) Inventors:
• **CAMMAROTA, Giovanni**
**00168 Rome (IT)**
• **MASUCCI, Luca**
**00168 Rome (IT)**

(74) Representative: **Predazzi, Valentina et al Società Italiana Brevetti S.p.A.**
**Piazza di Pietra, 39**
**00186 Roma (IT)**

(56) References cited:
**EP-A1- 0 856 259      WO-A1-03/071883**
**US-A1- 2012 315 249   US-A1- 2014 079 676**

• **"Nexabiotic Compare Probiotics", INTERNET CITATION, 1 January 2011 (2011-01-01), pages 1 - 9, XP009516416, Retrieved from the Internet <URL:https://nexabiotic.com/compareprobiotics >**
• **ANONYMOUS: "DrFormulas Natural Probiotics Nexabiotic Advanced with L. acidophilus", 5 April 2019 (2019-04-05), XP055691709, Retrieved from the Internet <URL:https://web.archive.org/web/20190405124 351/https://drformulas.com/products/nexabiotic -23-strain-probiotics-with-bifidobacterium-infan tis-saccharomyces-boulardii-lactobacillus-acid ophilus> [retrieved on 20200505]**

EP 4 009 995 B1

- ANONYMOUS: "UEG - United European Gastroenterology", 1 January 2018 (2018-01-01), XP055691537, Retrieved from the Internet <URL:https://ueg.eu/library/a-culturomics-based-synthetic-microbiota-consortium-derived-from-successful-bacterial-engrafters-is-a-safe-and-effective-treatment-for-recurrent-clostridium-difficile-infection-a-pilot-study/180227> [retrieved on 20200505]
- JAMES ROGER MCILROY ET AL: "Current and future targets for faecal microbiota transplantation", HUMAN MICROBIOME JOURNAL, vol. 11, 1 March 2019 (2019-03-01), pages 100045, XP055691538, ISSN: 2452-2317, DOI: 10.1016/j.humic.2018.08.004
- G. CAMMAROTA ET AL: "Randomised clinical trial: faecal microbiota transplantation by colonoscopy vs. vancomycin for the treatment of recurrent Clostridium difficile infection", ALIMENTARY PHARMACOLOGY & THERAPEUTICS., vol. 41, no. 9, 1 March 2015 (2015-03-01), GB, pages 835 - 843, XP055691533, ISSN: 0269-2813, DOI: 10.1111/apt.13144
- E.O. PETROF ET AL: "Microbial ecosystems therapeutics: a new paradigm in medicine?", BENEFICIAL MICROBES, vol. 4, no. 1, 1 March 2013 (2013-03-01), NL, pages 53 - 65, XP055691542, ISSN: 1876-2883, DOI: 10.3920/BM2012.0039
- ELAINE O PETROF ET AL: "Stool substitute transplant therapy for the eradication of Clostridium difficile infection: 'RePOOPulating'", MICROBIOME, BIOMED CENTRAL LTD, LONDON, UK, vol. 1, no. 1, 9 January 2013 (2013-01-09), pages 3, XP021138289, ISSN: 2049-2618, DOI: 10.1186/2049-2618-1-3
- MING LI ET AL: "Fecal microbiota transplantation and bacterial consortium transplantation have comparable effects on the re-establishment of mucosal barrier function in mice with intestinal dysbiosis", FRONTIERS IN MICROBIOLOGY, vol. 6, 7 July 2015 (2015-07-07), XP055691546, DOI: 10.3389/fmicb.2015.00692

Remarks:
The file contains technical information submitted after the application was filed and not included in this specification

(52) Cooperative Patent Classification (CPC): (Cont.)

C-Sets
A61K 35/741, A61K 2300/00;
A61K 35/742, A61K 2300/00;
A61K 35/744, A61K 2300/00;
A61K 35/745, A61K 2300/00;
A61K 35/747, A61K 2300/00

**Description**

STATE OF THE PRIOR ART

The infection by *Clostridium difficile* (CDI), an anaerobic non-spore-forming bacterium, can cause diarrhea and colitis, with severe complications such as megacolon, intestinal perforation, shock and death. Intestinal colonization occurs by fecal-oral route and is fostered by modification of the intestinal flora following a preceding antibiotic therapy. After the colonization, CD produces toxins (A and B) which bind to receptors of cells of the intestinal mucosa, causing phlogosis and diarrhea. Inpatients, above all ≥65 y. o. ones, in hospital environments, are particularly exposed to CD infection. The standard treatment, though effective, does not eliminate the risk of a high rate of infection recurrence (equal to 35% of which 40-45% encounters a second recurrence; after 2 or 3 recurrences, 60-65% of patients undergoes multiple recurrences), by "recurrence" meaning the reappearance of symptoms and the toxin positivity in feces within a month from the end of treatment, owing to the persistence of spores or of reinfection. In the last decade, the problems linked to CDI have increased owing to a greater diffusion in a nosocomial environment and to the appearance of new, more virulent strains.

The standard first-line treatment envisages the possibility of using vancomycin or metronidazole with a 97-87% eradication rate, respectively. However, after an initial therapeutic success, up to the 35% of all treated patients encounter a first recurrence of the infection, as a consequence of the persistence of spores or of reinfection. To treat recurrent CDI, novel therapeutic approaches envisaging the use of rifaximin, of nitazoxanide, or of fidaxomicin were introduced.

However, in an age of "antibiotic stewardship", i.e. a set of coherent actions promoting the responsible use of antibiotics, the need for means alternative to antibiotics in the treatment and in the prevention of infectious pathogens, in order to avoid the development of spreadable multidrug-resistant germs, is ever more felt.

To this end, immunological therapies such as the intravenous administration of immunoglobulins or of monoclonal antibodies specific against *Clostridium difficile* (CD) toxin were developed.

Moreover, against *C. difficile* infection the transplantation of intestinal microbiota (or fecal transplantation) obtained from healthy donor proved very useful and decisive, allowing to obtain a decrease in deaths and colon resection surgery.

Said technique has been known in the literature since 1958, and since then an ever-increasing number of evidence demonstrated the effectiveness of fecal transplantation in the treatment of recurrent CD infection (CDI), so much that said procedure was introduced in U.S. and European guidelines as a therapeutic option for CDI treatment (Surawicz et al. - Am J Gastroenterol 2013; Debast et al. - Clin Infect Dis 2014) and was validated by the National Italian Transplant Centre, with numerous attention levels and structural limitations. Even though the action mechanism of the fecal transplantation is still unclear, it is plausible that the reconstitution and the function of the intestinal microbiota be of primary importance, as it constitutes a decisive factor of resistance against CD and other pathogens, through mechanisms including resistance to bacterial colonization and stimulation of the intestinal immune system. In particular, resistance to pathogen colonization includes production of antimicrobial factors and competition for nutrients and for receptor sites on the epithelium by the resident microbiota. The latter "instructs" and "triggers" the mucosal immune response required for maintaining the homeostasis of the commensal microbiota, by eliminating the damaging pathogens. The destruction of the normal intestinal microbial flora, also known as dysbiosis, can be caused by infectious pathogens and by the use of antibiotics, as occurs in patients suffering from CDI.

Cammarota G., Masucci L. et al. ("Randomised clinical trial: faecal microbiota transplantation by colonoscopy vs. vancomycin for the treatment of recurrent Clostridium difficile infection", Aliment Pharmacol Ther. 2015 May;41(9):835-43.) have documented the effectiveness of said approach in 2015, in a controlled randomized clinical trial that compared fecal transplantation by colonoscopy (20 patients) with standard antibiotic therapy with vancomycin (19 patients) in patients with recurrent CDI, with results of 90% and 26% of infection eradication, respectively. During the clinical activity of intestinal microbiota transplantation for recurrent CDI, conducted by Cammarota and Masucci, numerous bacterial species were isolated and cultured from the feces of donors who had carried out previous donations of feces for intestinal microbiota transplantation with a successful outcome.

Among those, 15 were selected for the formulation of a bacterial "consortium". The 15 strains were selected by also assessing sensitivity to antibiotics, by international and certified reference tests, in order to avoid infusing any Multi-Drugs-Resistance microorganisms in receiving patients.

Moreover, additional studies demonstrated the stability of some bacterial species (*Bifidobacterium* genus, *Bacteroides* and *Clostridium* coccoid groups) up to six months from fecal transplantation.

However, fecal transplantation entails various drawbacks. In fact, to establish his/her suitability, the donor has to be subjected to a series of screening investigations overlappable to those carried out for a blood or organ donor, in order to guarantee the non-transmissibility of any pathogens to the receiver. This, of course, entails a lengthening of the times and remarkable costs. In studies published to date, different methods of microbiota infusion were used, ranging from fecal infusion into the duodenum through a nasogastric-duodenal gavage, to colonoscopy, to fecal enemas, all invasive maneuvers with the need of medical devices and admission to hospital environments. Moreover, the preparation of the infusion requires the operativeness of a skilled laboratory technician, preferably a microbiologist. Besides, once the donor is deemed suitable, on the day of the donation the treatment of donated feces has to be finalized within six hours and has to be again analyzed with rapid molecular tests for the exclusion of any pathogens appeared in the last days. Said tests however cannot rule out *a priori* the presence of multidrug-resistant opportunistic pathogens.

Hence, to date this decisive and life-saving practice, not envisaging the use of antibiotics, is not widespread, precisely due to its scarce reproducibility, the need of facilities such as an adequate microbiology laboratory and endoscopy service, of skilled staff, of wards suitable for the management of complex and fragile patients, etc.

This has anyhow set the foundations for subsequent studies in order to further explore the technique on animal models, in which it a "consortium" of beneficial bacteria, isolated from healthy animals, was identified, which proved as effective as fecal transplantation in the healing of mice suffering from dysbiosis (Li M, et al. "Fecal microbiota transplantation and bacterial consortium transplantation have comparable effects on the re-establishment of mucosal barrier function in mice with intestinal dysbiosis", Front Microbiol. 2015 Jul 7;6:692). Therefore, it was deemed that the identification and the subsequent administration of a "consortium" of beneficial bacteria could make the technique of intestinal microbiota transplantation significantly more reproducible. The most relevant and "pioneering" document on said approach is Petrof et al. "Stool substitute transplant therapy for the eradication of Clostridium difficile infection", Microbiome. Jan 9;1(1):3. In 2013, these researchers treated two patients suffering from CDI with a bacterial consortium consisting of 33 strains obtained from donor patients, with symptomatology resolution and a demonstrated rebalancing of the patients' intestinal microbiota.

The use of probiotics, in patients commencing antibiotic therapy, for the prevention of CDI onset is documented in the literature. The results obtained indicate a 60.5% reduction (59.5% in adults; 69.4% in children; 61% in hospitalized patients; 69.4% in non-hospitalized patients) of the onset of CD-associated infections. However, contrasting results were obtained and no clinical trials with standardization of posology and probiotic strains used exist. (Lau CS, Chamberlain RS. "Probiotics are effective at preventing Clostridium difficile-associated diarrhea: a systematic review and meta-analysis". Int J Gen Med. 2016 Feb 22;9:27-37.). Already from 2008, during YALE UNIVERSITY WORKSHOP on PROBIOTIC RECOMMENDATIONS it emerged that all studies with a positive outcome on probiotics use in CDI did not support clear recommendations as to specific dosages and the strains used. Therefore, from a commercial standpoint there are no formulations structured for CDI treatment and prevention. (Spinler JK, Ross CL, Savidge TC. Probiotics as adjunctive therapy for preventing Clostridium difficile infection - What are we waiting for? Anaerobe. 2016 Oct;41:51-57. doi: 10.1016/j.anaerobe.2016.05.007.) To date, on the market there is only one generic formulation that was used, aside from in CDI prevention, in CDI treatment as well, but anyhow not specifically formulated. (Lifeway kefir) (Bakken JS. Staggered and tapered antibiotic withdrawal with administration of kefir for recurrent Clostridium difficile infection. Clin Infect Dis. 2014 Sep 15;59(6):858-61.

Among probiotic products for oral use there is, for example, Nexabiotic advanced probiotics contains 23 priobiotic stranis comprising Saccharomyces boulardii, Bifidobacterium infantis, and Lactobacillus acidophilus + 20 other strains, the product is idnicated as a probiotic that helps to helps restore digestive balance, improve weight loss, boost immune system, and enhance beautiful skin.

Petrof et al "Microbial ecosystem therapeutics: a new paradigm in medicine?" BENEFICIAL MICROBES Vol 4, no. 1, 1 march 2013, pages 53-65 reviews refined microbial ecosystem replacement therapies for use in the treatment of gut dysbiosis.

Hence, there still is a need for developing alternative therapeutic solutions effective in the prevention and/or in the treatment of intestinal diseases such as, by way of example, infectious and antibiotic therapy-associated diarrheas, e.g. those from *C. Difficile,* or intestinal dysbiosis, that be reproducible and capable of overcoming the drawbacks of those known in the art.

## SUMMARY OF THE INVENTION

The Inventors of the present invention have presently selected specific probiotic microorganisms, authorized by EFSA (European Food Safety Authority), carrying out a mixture particularly effective in the restoration of the bacterial flora of the human host, simulating a quota of the intestinal bacteria. Such a "consortium" of probiotics can be taken orally, in amounts such as to be able to reach the colon, solving all of the above-mentioned problems, associated with the infusion of intestinal microbiota and/or with the probiotic compositions known in the art.

The present invention therefore relates to a probiotic mixture consisting of, *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus;* a composition comprising said mixture and suitable excipients and/or additives, as well as food products comprising said mixture and suitable excipients and/or additives.

Moreover, the present invention also relates to said mixtures, compositions and products for use in the prevention and/or treatment of intestinal diseases selected among infectious and antibiotic therapy-associated diarrheas, e.g., those from *C. difficile,* or intestinal dysbiosis as defined in the claims and therefore suitable for, a preventative and/or therapeutic treatment of intestinal diseases selected among infectious and antibiotic therapy-associated diarrheas, e.g., those from *C. difficile;* or intestinal dysbiosis, comprising the administration in therapeutically effective doses of said mixture, composition or food product.

Advantageously, the administration by oral route of the mixtures or of the compositions of the invention allows to facilitate the treatment of intestinal diseases in patients suffering from colon pathologies, such as severe diverticulosis or colon strictures, who, being unfit for colonoscopy treatments, might not benefit from intestinal microbiota transplantation.

DETAILED DESCRIPTION OF THE FIGURES

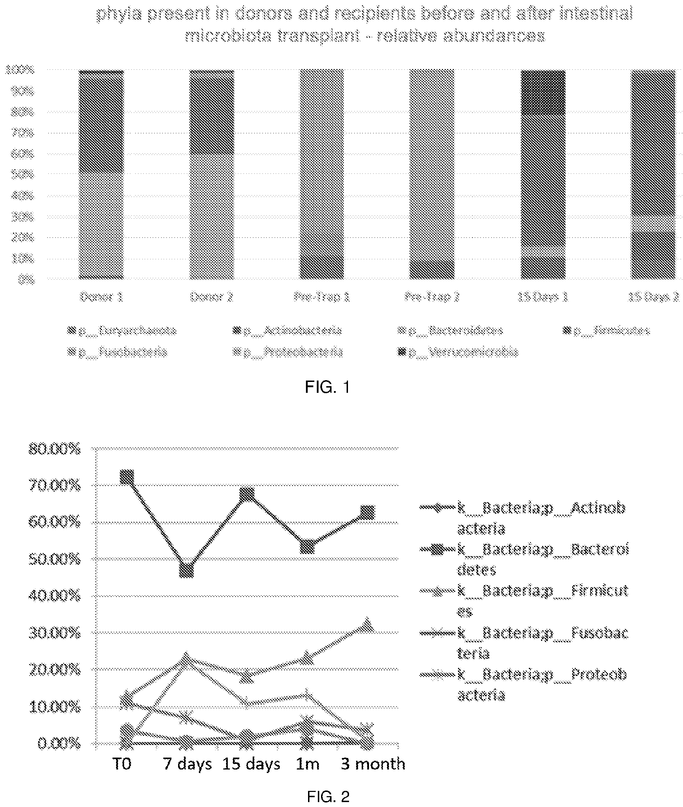

FIG. 1: the graph shows the relative abundance of the phyla present in donor and receiving patients before and after intestinal microbiota transplantation.

FIG. 2: the graph shows the time pattern of the phyla related to patient 1. Before receiving the infusion (T0) the patient had an intestinal microbiota fostering *Bacteroidetes* Phylum (72%). *Firmicutes* and *Proteobacteria* were present with a relative abundance respectively of 12% and 10%. *Verrucomicrobia* were present at 0.01%. At time T1 (7 days) *Bacteroidetes* level drops to 47% with an increase of *Firmicutes* to 23% and *Verrucomicrobia* to 22%.

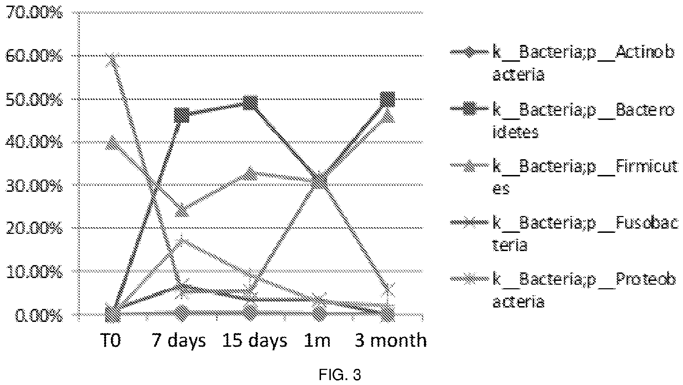

FIG. 3: the graph shows the time pattern of the phyla related to patient 2. Before receiving the infusion (T0) the intestinal bacterial population appeared consisting of *Proteobacteria* (60%), *Firmicutes* (40%) and with a reduction of *Bacteroidetes* (0.05%) and *Verrucomicrobia* (0.02%) Phyla. In subsequent follow-ups *Proteobacteria* reach 5%, whereas *Bacteroidetes* Phylum reaches 50% and *Verrucomicrobia* Phylum 17%. At +3 month post-infusion the microbiota seems to be stable.

GLOSSARY

In the context of the present description, the terms "probiotic" and "probiotic species" have the meaning commonly used in the literature and indicate, according to the official definition by FAO and OMS, "live microorganisms which when administered in adequate amounts confer a health benefit on the host". Specifically, in the present description, these terms refer to microorganisms used as food supplement or complement.

In the context of the present description, the wording "probiotic consortium" is equivalent to a "probiotic mixture" and means association of specific probiotic species.

In the context of the present description, the wording "human intestinal microbiota" has the meaning commonly used in the literature and means set of commensal microorganism (e.g., intestinal bacteria, viruses, fungi and protozoa) which coexist with the human organism without damaging it.

In the context of the present description, the wording "effective amount" means amount of active agent, or of composition comprising the active agents or of product comprising the active agents, sufficient to cause the desired benefits and, at the same time, low enough so as not to entail serious side effects.

In the context of the present description, "about" refers to the experimental error that can occur during conventional measurements. More particularly, when referring to a value it indicates ± 5% of the indicated value, and, when referring to a range, ± 5% of the extremes thereof.

DETAILED DESCRIPTION OF THE INVENTION

The Authors of the present invention have selected a probiotic mixture, consisting of *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus* particularly effective in the prevention and/or in the treatment of intestinal diseases selected from infectious and antibiotic therapy-associated diarrheas, e.g., those from *C. difficile,* or from intestinal dysbiosis.

Hence, the present invention refers to a probiotic mixture consisting of, the following probiotic species: *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus.*

In one embodiment, the present invention refers to a probiotic mixture consisting of, at least one strain of each of the following probiotic species: *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus.*

In one preferred embodiment, the probiotic mixture comprises *Bifidobacterium bifidum, Bifidobacterium breve,*

*Bifidobacterium longum, Bacillus subtilis, Lactobacillus casei, Lactobacillus gasseri, Lactobacillus rhamnosus, Enterococcus faecium* and *Streptococcus thermophilus* as sole microorganisms active in the prevention and/or in the treatment of intestinal diseases.

In one embodiment of the invention the above-described probiotic mixture is combined with inulin.

In particular, such intestinal affections are infectious and antibiotic therapy-associated diarrheas, caused by alteration of the intestinal bacterial flora in general. Said alteration can be a generic alteration of the intestinal bacterial flora of an individual, also known as intestinal dysbiosis, or can also be worsened or caused by infections from pathogenic microorganisms, such as, e.g., *C. difficile* infections.

The mixture of the invention may therefore consist in the association of one or more strains of the above-listed bacteria, or may comprise other compounds (active and non-active) on condition that said compounds be not principles or compounds pharmacologically active in the prevention and/or in the treatment of intestinal diseasesselected among infectious and antibiotic therapy-associated diarrheas, e.g., those from *C. difficile,* or intestinal dysbiosis.

Said compounds can be, e.g., pharmaceutical active principles for the treatment of associated pathologies, or can be one or more prebiotics fostering the colonization of the intestinal mucosa by the probiotic bacteria present in the mixture.

Hereinafter, the probiotics selected by the Authors of the present invention are briefly described.

Bifidobacteria are Gram-positive pleomorphic, non-spore-forming, anaerobic bacilli, belonging to *Actinobacteria* Phylum. *Bifidobacterium* genus is the main colonizer of humans and a remarkable importance thereof since birth is recognized. *Bifidobacterium bifidum, Bifidobacterium breve* and *Bifidobacterium longum* are the main colonizers of infants, and gradually over the years the first two abate, giving space to the proliferation of *B. catenulatum* and *B. adolescentis,* but the presence of *B. longum* remains. This change is likely to occur due to the modification of alimentary and environmental habits.

*Bifidobacteria* ability to stimulate the immune system, above all that of balancing the T-helper 1(TH1)/TH2 ratio in newborns, is demonstrated to such an extent that their use is widespread in the prevention of intestinal diseases, diarrheas, even necrotizing ones, or systemic diseases like allergies, celiac disease, obesity. Moreover, their effectiveness is proven. The main stimulators of the immune system are *B. bifidum* and *B. longum.*

*Bacillus subtilis, Firmicutes* Phylum, is a Gram-positive anaerobic spore-forming microorganism. Its spore has the same peculiarities of *C.difficile,* though differing by 75% in protein content. Due to this feature, it is capable of surviving gastric pH and also of reaching the large intestine. Moreover, it is recognized to have positive effects through the production of amino acids, enzymes and antimicrobial components such as bacteriocins, with remarkable effects on Gram + and Gram - enteropathogens. In particular, *B.subtilis* has a remarkable stimulating effect on Gut-Associated Lymphoid Tissue -GALT. Another positive feature is that *B. subtilis,* used as a probiotic, adheres to intestinal (gut) cells and, through an environmental competition mechanism, fights intestinal pathogens.

Lactobacilli are Gram-positive aerobic, facultative anaerobic bacteria belonging to *Firmicutes* Phylum. The *Lactobacillus* genus is important for maintaining the stability of the gastrointestinal tract, preventing intestinal infections and interacting with intestine epithelial cells. Lactobacilli are usually vehicled by milk and dairy products. For this reason, their non-pathogenicity and their peculiarity of resisting the action of bile has been known longtime. Moreover *Lactobacillus* spp. produce bactericidal substances such as bacteriocins, hydrogen peroxide and organic acids, which control the growth of pathogenic bacteria, both Gram-positive and Gram-negative ones.

The demonstrated beneficial activity of these probiotics and their adaptability to various foodstuffs have helped to introduce them in different vehicles other than milk, such as fruit juices or tomato extracts, indicated as supplements in vegan diets. In particular: *Lactobacillus rhamnosus* rebalances autochtonous bifidobacteria, reduces the inflammatory stage through P40 protein production; inhibits intestinal apoptosis, is capable of stimulating cytokines (interleukins, gamma Interferon, Tumor Necrosis Factor) and produces a bioprotective film; *Lactobacillus* casei produces Vitamin B-12; *Lactobacillus gasseri* is among the ones most isolated from human feces.

*Enterococcus faecium* is a Gram-positive coccus belonging to *Firmicutes* Phylum. Its use as a probiotic is specifically linked to the anti-diarrhea action (against both infective and antibiotic-correlated diarrheas). *E. faecium* is a commensal of the intestinal tract and this factor seems to positively influence its action, as well as the fact of having a rapid adaptability to the environment (lag-phase) and reduced replication. Its inhibition on the growth of enteropathogens such as *Escherichia coli, Salmonella serovars, Shigella spp.* and *Enterobacter spp.* is demonstrated, and, alike the other probiotics, it is resistant to an acidic pH and to bile.

To guarantee the safety of the probiotic strain, the IS16, hylEfm, esp, complete genome sequence and MIC (Minimum Inhibitory Concentration) to various antibiotics, ampicillin included, (Antimicrobial Resistance - AMR) parameters should be considered.

*Streptococcus thermophilus,* a Gram-positive coccus belonging to *Firmicutes* Phylum, is one of the main thermophilic homofermentative bacteria, widely used in the dairy industry. It produces an hexopolysaccharide that has proven antiinflammatory probiotic properties such as to address its study toward development and use as antioxidant and anticarcinogenic.

In one embodiment, the mixture may comprise a specific strain of each of the different probiotic species making up the above-mentioned mixture.

However, for the purpose of the present invention, any known strain of each of the probiotic species making up the mixture according to any one of the embodiments described herein may be used. Moreover, all species and the selected strains are commercially available and anyhow can be isolated according to the techniques known to a person skilled in the art.

Merely by way of example and not for limitative purposes, the mixture may comprise *Bifidobacterium bifidum* strain code BBF210 (BBF055) EU COLLECTION LMG P-29508 (DSM25565) ROELMI-HPC. Merely by way of example and not for limitative purposes, the mixture may comprise *Bifidobacterium breve* strain code PBS077 EU COLLECTION (DSM25173) ROELMI-HPC. Merely by way of example and not for limitative purposes, the mixture may comprise *Bifidobacterium longum* strain code BLG240 (PBS108) EU COLLECTION LMG P-29511 (DSM25174) ROELMI-HPC. Merely by way of example and not for limitative purposes, the mixture may comprise *Bacillus subtilis* strain code SNZ-1972 *Azienda Chimica e Farmaceutica.* Merely by way of example and not for limitative purposes, the mixture may comprise *Lactobacillus casei* strain code LC010 EU COLLECTION (DSM25569) ROELMI-HPC. Merely by way of example and not for limitative purposes, the mixture may comprise *Lactobacillus gasseri* strain code LG050 EU COLLECTION LMG P-29638 ROELMI-HPC. Merely by way of example and not for limitative purposes, the mixture may comprise *Lactobacillus rhamnosus* strain code LRH020 EUCOLLECTION LMG P-29513 (DSM25568) ROELMI-HPC. Merely by way of example and not for limitative purposes, the mixture may comprise *Enterococcus faecium* strain code LMG S-28935 EUCOLLECTION LMG P-21906 ROELMI-HPC. Merely by way of example and not for limitative purposes, the mixture may comprise *Streptococcus thermophilus* strain code ST075 EUCOLLECTION (DSM26721) ROELMI-HPC.

In one embodiment, the present invention refers to a probiotic mixture as defined in the claims, comprising, or consisting of, at least one of the following bacterial strains: *Bifidobacterium bifidum* BBF210 (BBF055), *Bifidobacterium breve* PBS077, *Bifidobacterium longum* BLG240, *Bacillus subtilis* SNZ-1972, *Lactobacillus casei* LC010, *Lactobacillus gasseri* LG050, *Lactobacillus rhamnosus* LRH020, *Enterococcus faecium* LMG S-28935 and *Streptococcus thermophilus* ST075.

Though not a fundamental feature for the purposes of the therapeutic effect, the Inventors of the present invention have surprisingly found that, when in the probiotic mixture the facultative aerobic-anaerobic bacteria (*Lactobacillus casei, Lactobacillus gasseri, Lactobacillus rhamnosus, Enterococcus faecium* and *Streptococcus thermophilus*) are present at about 40 ± 5% and the anaerobic bacteria (*Bifidobacterium bifidum, Bifidobacterium breve, Bifidobacterium longum, Bacillus subtilis*) are present at about 60 ± 5%, of the total colony-forming units per gram (CFUs x g) of the probiotic mixture, the probiotic mixture of the present invention is capable of performing its beneficial effects in an optimum manner. This because, though not wishing to be bound by any specific theory, the physiological intestinal microbiota is mainly comprised of anaerobic bacteria.

Hence, in one preferred embodiment, in the mixture of the present invention 40 ± 5% is represented by facultative aerobic-anaerobic bacteria (*Lactobacillus casei, Lactobacillus gasseri, Lactobacillus rhamnosus, Enterococcus faecium* and *Streptococcus thermophilus)* and 60% ± 5% by anaerobic bacteria (*Bifidobacterium bifidum, Bifidobacterium breve, Bifidobacterium longum, Bacillus subtilis, Lactobacillus casei, Lactobacillus gasseri, Lactobacillus rhamnosus).*

The various probiotic species characterizing the mixture of the present invention can be present in a variable amount.

However, the Inventors have also found that when in the mixture of the present invention the probiotics of the *Bifidobacterium* genus (group 1: *Bifidobacterium bifidum, Bifidobacterium breve, Bifidobacterium longum*) are overall present at 50 ± 5%, the probiotics of the *Lactobacillus* genus (group 2: *Lactobacillus casei, Lactobacillus gasseri, Lactobacillus rhamnosus*) are overall present at 16.5% ± 5%, the probiotics *Enterococcus faecium* and *Streptococcus thermophilus* (group 3) are overall present at 23.5% ± 5% , and the probiotic *Bacillus subtilis* is present at 10% ± 5% of the total colony-forming units per gram (CFUs x g) of the probiotic mixture, the individual species making up each group are able to compensate each other, regardless of their amount. This means that, in one embodiment, the concentration of the individual species of each group can be freely changed, provided that the overall concentrations of the groups as abovedefined be maintained.

Hence, the present invention also refers to a probiotic mixture consisting of, 50% ± 5% of probiotics of the *Bifidobacterium* genus, 16.5% ± 5% of probiotics of the *Lactobacillus* genus, 23.5% ± 5% of *Enterococcus faecium* and *Streptococcus thermophilus* and 10% ± 5% of *Bacillus subtilis* of the total colony-forming units per gram (CFUs x g) of the probiotic mixture.

In other words, according to one embodiment of the present invention, in the probiotic mixture probiotics of the *Bifidobacterium* genus are present at 50% ± 5%, probiotics of the *Lactobacillus* genus are present at 16.5% ± 5%, *Enterococcus faecium* and *Streptococcus thermophilus* are overall present at 23.5% ± 5% and *Bacillus subtilis* is present at 10% ± 5% of the total colony-forming units per gram (CFUs x g) of the probiotic mixture.

Table 1: examples of dose variability of the individual probiotic species inside each group.

| | Probiotic | elective dose (%) | variable dose 1 (%) | variable dose 2 (%) |
|---|---|---|---|---|
| Group 1 | Bifidobacterium bifidum | 13.03 | 13.5 | 12 |
| | Bifidobacterium breve | 12.3 | 13.2 | 13.1 |
| | Bifidobacterium longum | 24.19 | 23 | 24.6 |
| Subtotal | Subtotal | 49.52 | 49.7 | 49.7 |
| Group 2 | Lactobacillus casei | 6.1 | 5.2 | 6.8 |
| | Lactobacillus gasseri | 4.1 | 4.2 | 3.9 |
| | Lactobacillus rhamnosus | 6.1 | 6.9 | 6.5 |
| Subtotal | | 16.3 | 16.3 | 17.2 |
| Group 3 | Enterococcus faecium | 12.04 | 11.4 | 11.5 |
| | Streptococcus thermophilus | 12.04 | 12.5 | 12 |
| Subtotal | | 24.08 | 23.9 | 23.5 |
| | Bacillus subtilis | 10.1 | 10.1 | 9.6 |
| Total | | 100 | 100 | 100 |

Moreover, the Inventors have also singled out concentration ranges within which each of said probiotic species performs its beneficial action in a more advantageous manner.

In particular, *Bifidobacterium bifidum* can be present in the mixture at 12%-14%, preferably at 13.03% of the total colony-forming units (CFUs) of the probiotic mixture. *Bifidobacterium breve,* can be present in the mixture at 11,6%-13,2%, preferably at 12.3% of the total CFUs of the probiotic mixture. *Bifidobacterium longum* can be present in the mixture at 22.8%-25.5%, preferably at 24.19% of the total CFUs of the probiotic mixture. *Bacillus subtilis* can be present in the mixture at 9.6%-10.7%, preferably at 10.1% of the total CFUs of the probiotic mixture. *Lactobacillus casei* can be present in the mixture at 5.2%-6.9%, preferably at 6.1% of the total CFUs of the probiotic mixture. *Lactobacillus gasseri* can be present in the mixture at 3.9%-4.2%, preferably at 4.1% of the total CFUs of the probiotic mixture. *Lactobacillus rhamnosus* can be present in the mixture at 5.2%-6.9%, preferably at 6.1% of the total CFUs of the probiotic mixture. *Enterococcus faecium* can be present in the mixture at 11.4%-12.5%, preferably at 12.04% of the total CFUs of the probiotic mixture. *Streptococcus thermophilus* can be present in the mixture at 11.4%-12.5%, preferably at 12.04% of the total CFUs of the probiotic mixture.

In one particularly preferred embodiment, the probiotic mixture of the present invention may consist of *Bifidobacterium bifidum* at 13.03%, *Bifidobacterium breve* at 12.3%, *Bifidobacterium longum* at 24.19%, *Bacillus subtilis* at 10.1%, *Lactobacillus casei* at 6.1%, *Lactobacillus gasseri* at 4.1%, *Lactobacillus rhamnosus* at 6.1%, *Enterococcus faecium* at 12.04% and *Streptococcus thermophilus* at 12.04% of the total colony-forming units of the probiotic mixture (see Table 2, reported herebelow).

The effective amount of CFUs of each probiotic of the mixture of the invention can vary depending on the final formulation.

In one embodiment, the mixture of the present invention preferably comprises $2.36 \times 10^{10}$ CFU/g $\pm$ $1.4 \times 10^9$ CFU/g of *Bifidobacterium bifidum*. In one embodiment, the mixture of the present invention preferably comprises $2.23 \times 10^{10}$ CFU/g $\pm$ $1.4 \times 10^9$ CFU/g of *Bifidobacterium breve.* In one embodiment, the mixture of the present invention preferably comprises $4.38 \times 10^{10}$ CFU/g $\pm$ $2.4 \times 10^9$ CFU/g of *Bifidobacterium longum.* In one embodiment, the mixture of the present invention preferably comprises $1.84 \times 10^{10}$ CFU/g $\pm$ $1 \times 10^9$ CFU/g of *Bacillus subtilis.* In one embodiment, the mixture of the present invention preferably comprises $1.1 \times 10^{10}$ CFU/g $\pm$ $1.5 \times 10^9$ CFU/g of *Lactobacillus casei.* In one embodiment, the mixture of the present invention preferably comprises $7.4 \times 10^{10}$ CFU/g $\pm$ $3 \times 10^8$ CFU/g of *Lactobacillus gasseri.* In one embodiment, the mixture of the present invention preferably comprises $1.1 \times 10^{10}$ CFU/g $\pm$ $1.5 \times 10^9$ CFU/g of *Lactobacillus rhamnosus.* In one embodiment, the mixture of the present invention preferably comprises $2.18 \times 10^{10}$ CFU/g $\pm$ $1 \times 10^9$ CFU/g of *Enterococcus faecium.* In one embodiment, the mixture of the present invention preferably comprises $2.18 \times 10^{10}$ CFU/g $\pm$ $1 \times 10^9$ CFU/g of *Streptococcus thermophilus.*

In another embodiment, the mixture of the present invention comprises $2.36 \times 10^{10}$ CFU/g $\pm$ $1.4 \times 10^9$ CFU/g of *Bifidobacterium bifidum,* $2.23 \times 10^{10}$ CFU/g $\pm$ $1.4 \times 10^9$ CFU/g of *Bifidobacterium breve,* $4.38 \times 10^{10}$ CFU/g $\pm$ $2.4 \times 10^9$ CFU/g of *Bifidobacterium longum,* $1.84 \times 10^{10}$ CFU/g $\pm$ $1 \times 10^9$ CFU/g of *Bacillus subtilis,* $1.1 \times 10^{10}$ CFU/g $\pm$ 1.5

x $10^9$ CFU/g of *Lactobacillus casei,* 7.4 x $10^{10}$ CFU/g $\pm$ 3 x $10^8$ CFU/g of *Lactobacillus gasseri,* 1.1 x $10^{10}$ CFU/g $\pm$1.5 x $10^9$ CFU/g of *Lactobacillus rhamnosus,* 2.18 x $10^{10}$ CFU/g $\pm$ 1 x $10^9$ CFU/g of *Enterococcus faecium* and 2.18 x $10^{10}$ CFU/g $\pm$1 x $10^9$ CFU/g of *Streptococcus thermophilus.*

In one embodiment of the invention, the mixture of the present invention is as reported in the following table (Table 2).

Table 2: Mixture of selected probiotics

| Probiotic | CFU/g | % |
|---|---|---|
| Bifidobacterium bifidum | 2.36 x $10^{10}$ CFU/g | 13.03% |
| Bifidobacterium breve | 2.23 x $10^{10}$ CFU/g | 12.3% |
| Bifidobacterium longum | 4.38 x $10^{10}$ CFU/g | 24.19% |
| Bacillus subtilis | 1.84 x $10^{10}$ CFU/g | 10.1% |
| Lactobacillus casei | 1.1 x $10^{10}$ CFU/g | 6.1% |
| Lactobacillus gasseri | 7.4 x $10^9$ CFU/g | 4.1% |
| Lactobacillus rhamnosus | 1.1 x $10^{10}$ CFU/g | 6.1% |
| Enterococcus faecium | 2.18 x $10^{10}$ CFU/g | 12.04 % |
| Streptococcus thermophilus | 2.18 x $10^{10}$ CFU/g | 12.04% |
| total | 1.81 x $10^{11}$ CFU/g | 100% |
| *CFU/g: colony-forming units per gram | | |

As mentioned above, the probiotic mixture described and claimed can advantageously be combined (e.g., mixed) with inulin.

The present invention also refers to a composition as defined in the claims, comprising as sole probiotics the bacteria of the probiotic mixture as described above, and optionally inulin and/or any formulation excipients.

Hence, the present invention also refers to a composition comprising a probiotic mixture of *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus* as sole probiotics, according to any one of the above-exemplified embodiments, and at least one suitable excipient and/or additive.

In one preferred embodiment, the composition of the invention also comprises inulin.

More particularly, the composition may comprise as sole probiotics, a probiotic mixture of: *Bifidobacterium bifidum BBF210 (BBF055), Bifidobacterium breve PBS077, Bifidobacterium longum BLG240, Bacillus subtilis* SNZ-1972, *Lactobacillus casei LC010, Lactobacillus gasseri LG050, Lactobacillus rhamnosus LRH020, Enterococcus faecium LMG S-28935, Streptococcus thermophilus ST075* and at least one suitable excipient and/or additive.

In one preferred embodiment, the composition of the invention also comprises inulin.

According to one embodiment of the invention, the composition may comprise sa sole probiotics, a probiotic mixture as described above, according to an overall dosage equal to 25 or 50 billions of live cells, and at least one suitable excipient and/or additive. Preferably, said composition also comprises inulin.

Said suitable excipient and/or additive and/or active principle can be a pharmaceutically acceptable excipient, and can be selected, e.g., among those generally known in the state of the art and includes, but is not limited to: carriers, fillers, humectants, disintegrating agents, binders, retarding agents, absorption accelerators, wetting agents, surfactants, adsorbents, lubricants, glidants, aromas, sweeteners and/or preservatives.

A person skilled in the art will know how to select suitable additives/excipients from the general knowledge in the field.

According to a preferred aspect of the invention, said composition also comprises inulin.

Hence, the present invention also refers to a composition comprising as sole probiotics a probiotic mixture of *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus* according to any one of the of the above-exemplified embodiments, and inulin.

In particular, said composition may contain inulin in an amount ranging from 20 and 60% by weight of said composition, e.g., for a dosage unit of 500 mg, said inulin can be from about 100 to about 300 mg.

In one preferred embodiment of the invention, the composition may comprise about the 25-30% by weight of inulin, e.g., for a dosage unit of 500mg, said composition may comprise about 136.15 ± 20 mg or 136.15 ± 30 mg of inulin, preferably 136.15 mg of inulin.

In a further preferred embodiment of the invention, said composition may comprise about 50-60% by weight of inulin, e.g., for a dosage unit of 500 mg, said composition may comprise about 268 ± 20 mg or 268 ± 30 mg of inulin, preferably 268 mg of inulin.

Therefore, for the carrying out of the invention suitable carriers, like, e.g., sodium citrate and calcium phosphate may be used. As fillers there may, e.g., be used, without however being limited to the examples provided, starch, lactose, microcrystalline cellulose, sucrose, glucose, mannitol, triglycerides and colloidal silica. Anti-agglomerating agents suitable for the carrying out of the invention are magnesium salts of fatty acids. Humectants suitable for the carrying out of the invention may be, e.g., glycerol or other commonly used humectants. A non-limiting example of disintegrating agents is given by alginates, calcium carbonate, starches, starch, cellulose and polyvinylpyrrolidone derivatives, silicates and sodium carbonate. Among the possible suitable binders there are, without however being limited thereto, carboxymethyl cellulose, alginates, gelatine, polyvinylpyrrolidone, sucrose, polymeric derivatives of cellulose, starch derivatives. Retarding agents suitable for the carrying out of the composition of the invention are, e.g., paraffin, cellulose polymers, fatty acid esters and the like. Suitable absorption accelerators may be, e.g., quaternary ammonium compounds. Among the wetting agents and surfactants suitable for the carrying out of the composition of the invention there are, e.g., cetyl alcohol and glycerol monostearate. A non-limiting example of adsorbents comprises bentonite clays and kaolin. Lubricants commonly used in the field are represented, e.g., by talc, calcium stearate, magnesium stearate, polyethylene glycol, sodium lauryl sulfate, sodium stearyl fumarate. Suitable glidants may be talc, colloidal silica. Aromas can be used when deemed necessary or when the formulation envisages them. Conventional sweeteners are represented by sucrose, fructose, xylitol, mannitol, sorbitol, stevioside, etc. Finally, as preservatives, e.g., benzoates, nitrites, sulfates and sorbates can be used.

The compositions according to any one of the embodiments provided in the present description may be formulated in any form, administered via any administration route and associated with any other component, in a variety of ways. The compositions are preferably, yet not exclusively, administered orally.

The compositions may be in a liquid, semiliquid, solid or semisolid form. Liquid forms suitable for an oral administration are, by way of example, drops, emulsions, solutions, suspensions (prepared or extemporary), syrups and elixirs. In one preferred embodiment, the compositions are solid formulations such as, e.g., tablets, hard or soft capsules, pills, gelatines, lozenges, powders, granules, sachets and films. The liquid or semiliquid formulations could be contained into suitable administration vectors.

The solid dosage forms, such as tablets, hard or soft capsules, gelatines, pills, lozenges, powders, granules, sachets and films, may also be coated with enteric, gastric coatings, or with coatings of other type known in the state of the art. They may be of the type enabling the release of active ingredients only or preferably in a certain intestinal tract, optionally in a delayed manner. Substances that can enable such a delayed use include, but are not limited to, polymers and waxes. In one preferred embodiment, the composition is formulated in dosage units in the form of a tablet or capsule.

A tablet or capsule suitable to be used for the oral administration of a composition subject-matter of the invention according to any one of the embodiments provided in the present description is any one acid-resistant, delayed-release (DR) tablet or capsule, suitable for fostering the release of active principles (agents) only or preferably inside the intestine, limiting instead the release thereof inside the stomach.

In one embodiment of the invention, said tablet or capsule containing a composition according to the invention is an acid-resistant, DR tablet or capsule, of natural and/or plant origin, such as, e.g., an hydroxypropylmethyl cellulose-based capsule. A formulation of plant origin is increasingly being sought by consumers, as it is suitable to all religious populations and to all lifestyles, and it is moreover without titanium dioxide, which has been demonstrated to be a promoter and aggravating factor of colitis.

According to a further aspect of the invention, said tablet or capsule does not contain gluten and/or lactose, and is therefore adapted to be taken also by celiac and/or intolerant subjects.

Examples of commercially available tablets or capsules that may be used for oral administration of a composition according to the invention, are the transparent acid-resistant DRcaps® capsules (Capsugel) of natural plant origin.

According to one embodiment of the invention, said capsule or tablet containing a composition according to any one of the variants described herein will have a total weight ranging from 300 and 600 mg, e.g. an overall weight equal to 300 mg, 400 mg, 500 mg, or 600 mg. In one preferred embodiment, said capsule or tablet containing a composition according to any one of the above-described embodiments, has an overall weight equal to 500 mg.

In one particularly preferred embodiment, the composition is formulated in the form of a gastro-resistant tablet or capsule according to the quali-quantitative composition reported in Table 3.

Table 3: colony-forming units per capsule of each of the selected probiotics.

| Probiotic (CFU/g of products used) | CFU/capsule | mg/dose |
|---|---|---|
| *Bifidobacterium bifidum* 250bn | $5.9 \times 10^9$ | 23.6 |
| *Bifidobacterium breve* 300bn | $5.6 \times 10^9$ | 18.58 |
| *Bifidobacterium longum* 100bn | $10.9 \times 10^9$ | 109.5 |
| *Bacillus subtilis* 100bn | $4.6 \times 10^9$ | 46 |
| *Lactobacillus casei* 300bn | $2.75 \times 10^9$ | 9.17 |
| *Lactobacillus gasseri* 200bn | $1.85 \times 10^8$ | 9.68 |
| *Lactobacillus rhamnosus* 300bn | $2.75 \times 10^9$ | 9.17 |
| *Enterococcus faecium* 500bn | $5.4 \times 10^9$ | 10.9 |
| *Streptococcus thermophilus* 200bn | $5.4 \times 10^9$ | 27.5 |
| | | mg/dose |
| inulin | | 136.15 |
| **Excipients** | | mg/dose |
| Magnesium salts of fatty acids | | 5 |
| gastro-resistant dr capsule | | 95 |
| TOTAL | | 500 |
| *The probiotics used have a specific yield per gram that can vary depending on producers. Hence, the quantitation in cfu/capsule and not in mg/dose applies. | | |

According to one embodiment, the present invention refers to a composition formulated in the form of a gastro-resistant tablet or capsule according to the quali-quantitative composition reported in Table 4.

Table 4: mean values of CFUs per capsule of each of the selected probiotics.

| Probiotic (cfu/g of product used) | CFU/capsule |
|---|---|
| *Bifidobacterium bifidum* DSM 25565 | $5.9 \times 10^9$ |
| *Bifidobacterium breve* DSM 25173 | $5.57 \times 10^9$ |
| *Bifidobacterium longum* DSM 25174 | $10.95 \times 10^9$ |
| *Bacillus subtilis* DSM 25565 | $4.6 \times 10^9$ |
| *Lactobacillus casei* DSM 25569 | $2.75 \times 10^9$ |
| *Lactobacillus gasseri* LMG P-29638 | $1.94 \times 10^9$ |
| *Lactobacillus rhamnosus* DSM 25568 | $2.75 \times 10^9$ |
| *Enterococcus faecium* LMG S-28935 | $5.45 \times 10^9$ |
| *Streptococcus thermophilus* DSM 26721 | $5.45 \times 10^9$ |
| | mg/dose |
| inulin | $136.15 \pm 20$ |
| **Excipients/additives** | mg/dose |
| Magnesium salts of fatty acids | q.s. |
| Hydroxypropylmethyl cellulose | q.s. |
| TOTAL | 500 |

One embodiment of the present invention refers to a composition formulated in the form of a gastro-resistant tablet or capsule according to the quali-quantitative composition reported in Table 5.

Table 5: mean values of CFUs per capsule of each of the selected probiotics.

| Probiotic (CFU/g of products used) | CFU/capsule |
|---|---|
| *Bifidobacterium bifidum* DSM 25565 | $2.95 \times 10^9$ |
| *Bifidobacterium breve* DSM 25173 | $2.8 \times 10^9$ |
| *Bifidobacterium longum* DSM 25174 | $5.4 \times 10^9$ |
| *Bacillus subtilis* DSM 25565 | $2.3 \times 10^9$ |
| *Lactobacillus casei* DSM 25569 | $1.4 \times 10^9$ |
| *Lactobacillus gasseri* LMG P-29638 | $0.98 \times 10^9$ |
| *Lactobacillus rhamnosus* DSM 25568 | $1.4 \times 10^9$ |
| *Enterococcus faecium* LMG S-28935 | $2.75 \times 10^9$ |
| *Streptococcus thermophilus* DSM 26721 | $2.7 \times 10^9$ |
| | **mg/dose** |
| inulin | 268 mg $\pm$ 20 |
| **Excipients/additives** | **mg/dose** |
| Magnesium salts of fatty acids | q.s. |
| hydroxypropylmethyl cellulose | q.s. |
| TOTAL | 500 |

According to one preferred embodiment, the present invention refers to a composition formulated in the form of a gastro-resistant tablet or capsule according to the quali-quantitative composition reported in Table 6.

Table 6: mean values of CFUs per capsule of each of the selected probiotics.

| Probiotic (CFU/g of products used) | CFU/capsule |
|---|---|
| *Bifidobacterium bifidum* DSM 25565 | $5.9 \times 10^9$ |
| *Bifidobacterium breve* DSM 25173 | $5.57 \times 10^9$ |
| *Bifidobacterium longum* DSM 25174 | $10.95 \times 10^9$ |
| *Bacillus subtilis* DSM 25565 | $4.6 \times 10^9$ |
| *Lactobacillus casei* DSM 25569 | $2.75 \times 10^9$ |
| *Lactobacillus gasseri* LMG P-29638 | $1.94 \times 10^9$ |
| *Lactobacillus rhamnosus* DSM 25568 | $2.75 \times 10^9$ |
| *Enterococcus faecium* LMG S-28935 | $5.45 \times 10^9$ |
| *Streptococcus thermophilus* DSM 26721 | $5.45 \times 10^9$ |
| | **mg/dose** |
| inulin | 136.15 |
| **Excipients/additives** | **mg/dose** |
| Magnesium salts of fatty acids | q.s. |
| hydroxypropylmethyl cellulose | q.s. |
| TOTAL | 500 |

According to one preferred embodiment, the present invention refers to a composition formulated in the form of a gastro-resistant tablet or capsule according to the quali-quantitative composition reported in Table 7.

Table 7: mean values of colony-forming units per capsule of each of the selected probiotics.

| Probiotic (CFU/g of the products used) | CFU/capsule |
|---|---|
| *Bifidobacterium bifidum* DSM 25565 | $2.95 \times 10^9$ |
| *Bifidobacterium breve* DSM 25173 | $2.8 \times 10^9$ |
| *Bifidobacterium longum* DSM 25174 | $5.4 \times 10^9$ |
| *Bacillus subtilis* DSM 25565 | $2.3 \times 10^9$ |
| *Lactobacillus casei* DSM 25569 | $1.4 \times 10^9$ |
| *Lactobacillus gasseri* LMG P-29638 | $0.98 \times 10^9$ |
| *Lactobacillus rhamnosus* DSM 25568 | $1.4 \times 10^9$ |
| *Enterococcus faecium* LMG S-28935 | $2.75 \times 10^9$ |
| *Streptococcus thermophilus* DSM 26721 | $2.7 \times 10^9$ |
| | **mg/dose** |
| inulin | 268 mg |
| **Excipients/additives** | **mg/dose** |
| Magnesium salts of fatty acids | q.s. |
| hydroxypropylmethyl cellulose | q.s. |
| TOTAL | 500 |

An error margin equal to $\pm 10^2$ or $\pm 10^3$ can be associated with all mean values of colony-forming units per capsule reported in the preceding Tables.

The formulations, according to any one of the embodiments described herein, may be prepared according to conventional methods known to a person skilled in the art. E.g., for the preparing of the foregoing capsule the selected probiotics could be mixed, and conventional excipients added up to the capsule volume, where the conventional excipients are as defined above.

Moreover, in accordance with any of the embodiments, the mixture or the composition of the present invention are mainly intended to be used by human beings, but can also be used on animals.

The present invention also refers to a probiotic mixture consisting of, *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus,* optionally combined with inulin, for use as a medicament.

In particular, the present invention also refers to a probiotic mixture consisting of, *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus,* optionally combined with inulin, for use in the prevention and/or treatment of intestinal diseases selected amon the infectious and antibiotic therapy-associated diarrheas, e.g., those from *C. difficile,* or intestinal dysbiosis.

Even more particularly, the present invention refers to a probiotic mixture consisting of *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus,* optionally combined with inulin, for use in the prevention and/or treatment of diarrhea from *C. difficile.*

The present invention also refers to a composition comprising as sole priobiotics, a probiotic mixture of *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus,* optionally combined with inulin, for use as a medicament.

In particular, the present invention also refers to a composition comprising as sole probiotics, a probiotic mixture of *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus,* optionally combined with inulin, for use in the prevention and/or treatment of intestinal diseases selected among the infectious and antibiotic therapy-associated diarrheas, e.g., those from *C. difficile,* and therefore the associated intestinal dysbiosis.

Even more particularly, the present invention refers to a composition comprising as sole probiotics the probiotic mixture of *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus,* optionally combined with inulin and any other formulation excipients for use in the prevention and/or treatment of diarrhea from *C. difficile* and antibiotic-associated diarrhea.

The present invention also refers to a food product comprising the probiotic mixture as described above and in the claims. Said food product may be, e.g., a food supplement, beverage, nutraceutical, aliment and functional or medicated aliment. Even more particularly, the food product, comprising the probiotic mixture of the invention, may be a yogurt, juice, a smoothie, extract, extracted juice, food bar, gelatine or the like.

Hence, the present invention also refers to a food product, be it a food supplement, beverage, nutraceutical, an aliment, a functional or medicated aliment, comprising the probiotic mixture of *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus* and at least one suitable excipient and/or additive, like, e.g., inulin and any formulation excipients, where the suitable excipient and additive are as defined above.

The advantages of the probiotic mixture of the present invention, and therefore of compositions or products containing it, compared to fecal transplantation and/or other probiotic compositions known in the art, will be apparent to a person skilled in the art. In particular, compared to fecal transplantation, there is no need anymore to select a donor with the involved problems of finding him/her and of the high costs; neither admission to hospital environments nor the use of medical devices are necessary; a laboratory for preparing the infusion is not necessary; higher safety, as validated and certified bacteria are used; it can be taken orally, and is therefore usable by all kids of patients, hospitalized and non-hospitalized ones. Moreover, compared to other probiotic compositions known in the art, it ensures higher reproducibility in the prevention and/or the treatment of intestinal affections.

Other advantages of the probiotic mixture of which to the present invention will be immediately evident to a person skilled in the art on the basis of the preceding description and of the examples reported hereinafter.

EXAMPLES

The examples reported hereinafter are merely by way of illustration and are not meant to limit the scope of the present invention. Variations and modifications of any of the embodiments described herein, which are obvious to a person skilled in the art, are encompassed in the object of the appended claims.

*Example 1: microbial load assessment in solution of feces from healthy donors of microbiota for fecal infusion.*

About 300 infusions of intestinal microbiota from healthy donors into CDI patients were carried out. Laboratory preparation consists in obtaining a fecal microbiota solution through various washing steps and filtrations. The number of bacteria present in the solutions was quantitated and the Inventors obtained non-constant bacterial concentrations, just due to the variables that may exist in the preparing of the infusion. Therefore, the mean bacterial value obtained was of $1.7 \times 10^{10}$ UFC/g of feces.

20 solutions for fecal infusion were quantitated. Starting from 1 ml of solution, nine 1:10 serial dilutions were performed, down to $10^{-9}$. The $10^{-9}$ dilution was cultured by using a 10 μl calibration loop, where grown bacterial colonies (CFUs) were detected.

Then, the following formula was used:

$$\frac{N \text{colonies}}{0.01 ml} * 10^9 * 1ml = CFU/ml$$

Then, by using the formulas:

$$\frac{(total\ feces)g}{(solution\ total\ volume)ml} = g\ (feces)/ml$$

$$CFU/g\ (feces) = \frac{1g}{g\ (feces) * ml} * CFU/ml$$

14

the Inventors obtained the CFU/g of feces.

*Example 2: assessment of phyla percentage in healthy donors and receivers.*

By pyrosequencing analysis, it was possible to single out how the donors of feces for fecal transplantation were equipped at the Phylum level with a characteristic intestinal microbiota. In fact, a majority of *Firmicutes* (45%-80%) and a minority of *Proteobacteria* (1-4%) were present. The receivers, suffering from CDI, exhibited instead an inverted *Proteobacteria* (50%-80%) and *Firmicutes* (10%-19%). microbiotic profile.

After infusion of the donor's intestinal microbiota, CDI patients' microbiotas exhibited a recovery of the profile toward a reacquiring of a right proportion of *Firmicutes* (FIG. 1). Hence, it was possible to verify that infusions by colonoscopy route of the mixture di probiotics of the invention have had a decisive success in CDI patients.

*Comparative example 3: comparison of the effectiveness of the 9-probiotic mixture of the invention compared to other mixtures.*

The 9-probiotic mixture of the invention was tested for the treatment of *C. difficile* infection, through administration by colonoscopy route, in comparison with the mixture of 15 bacterial strains (*Acidaminococcus intestinii, Alistipes finegoldii, Bacteroides fragilis, Bacteroides ovatus, Bacteroides uniformis, Bifidobacterium longum, Clostridium scindens, Escherichia coli, Lactobacillus casei, Lactobacillus gasseri, Lactobacillus rhamnosus, Lactobacillus parabuchnerii, Parabacteroides distasonis, Propionibacterium avidum, Ruminococcus gnavus*) experimented beforehand. Six patients were treated, three with the mixture of 15 bacterial strains and three with the mixture of 9 bacterial strains.

By pyrosequencing it was possible to verify two patients treated with the formulation of the 15 bacterial strains. In FIG. 2 the graph shows the time pattern of the Phyla related to patient 1. Before receiving the infusion (T0) the patient possessed an intestinal microbiota in favor of *Bacteroidetes* Phylum (72%). *Firmicutes* and *Proteobacteria* were present with a relative abundance respectively of 12% and 10%. *Verrucomicrobia* were present for 0.01%. At time T1 (7 days) the level of *Bacteroidetes* reduces to 47% with an increase of *Firmicutes* to 23% and *Verrucomicrobia* to 22%. In the subsequent follow-ups a maintaining of the balance of the intestinal microbiota is highlighted.

In patient 2 (FIG. 3) at T0 the intestinal bacterial population appeared comprised of *Proteobacteria* (60%), *Firmicutes* (40%) and a reduction of the *Bacteroidetes* (0.05%) and *Verrucomicrobia* (0.02%) Phyla. In the subsequent follow-ups, *Proteobacteria* reach 5%, whereas *Bacteroidetes* Phylum reaches 50% and *Verrucomicrobia* 17%. At month +3 post-infusion the microbiota seems to be stable.

From a clinical standpoint, the resolution of diarrhea and a negativization of toxins A/B of *C.difficile* was obtained in all patients enrolled.

*Example 3: assessment of the effectiveness of the probiotic mixture by oral route.*

The protocol of the experimental clinical trial envisages the administration of the mixture of the 9 bacterial strains through gastro-resistant capsules (two tablets/day two days per week and for 4 weeks) in patients suffering from CDI and post-antibiotic therapy diarrhea with placebo control arm.

In the experimental clinical trial gastro-resistant capsules having a composition as reported in the examples of Tables 6 and 7 are used.

In order to verify the effectiveness of the probiotic formulation, besides clinical assessment also the fecal samples of patients before the treatment and at + 15 days, were analyzed, by Next Generation Sequencing and the search for A/B toxins of *C.difficile* in all patients enrolled.

The preliminary results obtained highlighted the ability of gastro-resistant capsules containing the bacterial consortium subject-matter of the study to modulate the intestinal microbiota of the diseased subjects, fostering a resolution of the symptomatology in CDI patients.

The percentages indicated in the present description are to be understood as percentages by weight of the mixture or of the composition, depending on the context in which they are expressed.

The protocol of the experimental clinical trial set up to assess the effectiveness of the probiotic mixture by oral route is summarized herein as follows.

Experiments under way

The study is designed as a proof-of-concept prospective trial randomized and controlled, under blind conditions, for which 40 patients are enrolled.

Subjects in possession of all inclusion criteria are enrolled, and all subjects in possession of at least one exclusion criterion are excluded.

Inclusion criteria

- Patients with diarrhea subsequent to treatment with antibiotic therapies.
- Diarrhea of any degree, measurable according to Common Terminology Criteria (CTC) for Adverse Events (AE) version 4.0, onset after the antibiotic treatment.
- Absence of diarrhoeic symptomatology in the three months before the antibiotic treatment.
- Age > 18 years
- Ability to express consent to treatment

Exclusion criteria

- Any clinical condition that, in the experimenters' opinion, may contraindicate enrollment in the study (actual or previous neoplasia; cardiopathies; pneumological diseases);
- Oher causes of diarrhea overlappable to that caused by antibiotics (infections, intestinal chronic inflammatory diseases, irritable bowel syndrome, from drugs);
- Positivity of fecal parasitological examination for *Giardia lamblia, Entamoeba histolyticaldispar, Cryptosporidium parvum,* and other parasites;
- Coproculture positivity for Salmonella spp., *C. difficile, Shigella spp., Yersinia enterocolitica, Campylobacter, Streptococcus agalactiae, Staphylococcus aureus, Escherichia coli* enteropathogens and other microorganisms;
- Positivity of *C. difficile* toxin;
- Previous total or partial colectomy surgery;
- Patients with cutaneous enterostomy;
- Immunosuppressive therapy carried out 4 weeks before enrollment;
- Pregnancy or breastfeeding;
- Concomitant enrollment in other interventional experimental protocols;
- Unstable personality, or unable to comply with protocol procedure.

Treatments

All enrolled patients, that be in possession of all inclusion criteria and no exclusion criteria, are required to deliver a sample of feces before the start of the treatment. Subsequenty, patients are randomized (1:1, by statistical computer software), to one of the two treatment groups:

- Group A) Administration of bacterial consortium: 1 gastro-resistant capsule containing 50 billions (bn) bacteria, twice daily (at 10.00 and 17.00 hrs, respectively, on an empty stomach), for one week.
- Group B) Administration, with the same treatment modes of group A), of placebo-containing capsule.

All randomized patients (time zero [T0]) both in group A and in group B are required to deliver a sample of feces in T0 before treatment and 30 days after treatment, for the purposes of the characterization of the fecal microbiota after said treatment. First of all, patients' compliance with treatment is analyzed. For each population the effectiveness is assessed in terms of objective response, in particular of the reduction/resolution of diarrhea in the two treatment groups. Any difference is assessed according to the chi-squared method for unpaired data. The analysis is conducted with the Kaplan and Meier method. Differences between the two populations are analyzed with the Log-rank test. Moreover, fecal microbiota characterization is analyzed before and after the treatment. Microbiota analysis is carried out with metagenomic method (16S rRNA gene sequencing), after DNA and RNA extraction, sequencing included.

**Claims**

1. Probiotic mixture consisting of *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* and *Streptococcus thermophilus.*

2. Probiotic mixture according to claim 1, wherein facultative aerobic-anaerobic bacteria (*Lactobacillus casei, Lacto-*

*bacillus gasseri, Lactobacillus rhamnosus, Enterococcus faecium* and *Streptococcus thermophilus*) are present at 40 ± 5% and anaerobic bacteria (*Bifidobacterium bifidum, Bifidobacterium breve, Bifidobacterium longum* and *Bacillus subtilis,*) are present at 60 ± 5% of the total colony-forming units per gram (CFU x g) of the probiotic mixture.

3. Probiotic mixture according to claim 1 or 2, wherein probiotics of the genus *Bifidobacterium* are present at 50% ± 5%, probiotics of the genus *Lactobacillus* are present at 16.5% ± 5%, *Enterococcus faecium* and *Streptococcus thermophilus* are overall present at 23.5% ± 5% and *Bacillus subtilis* is present at 10% ± 5% of the total colony-forming units per gram (CFU x g) of the probiotic mixture.

4. Probiotic mixture according to any one of claims 1 to 3, wherein *Bifidobacterium bifidum* is present at 12%-14%, preferably at 13.03%; *Bifidobacterium breve* is present at 11.6%-13.1%, preferably at 12.3%; *Bifidobacterium longum* is present at 22.8%-25.5%, preferably at 24.19%; *Bacillus subtilis* is present at 9.6%-10.7%, preferably at 10.1%; *Lactobacillus casei* is present at 5.2%-6.9%, preferably at 6.1%; *Lactobacillus gasseri* is present at 3.9%-4.2%, preferably at 4.1%; *Lactobacillus rhamnosus* is present at 5.2%-6.9%, preferably at 6.1%; *Enterococcus faecium* is present at 11.4%-12.5%, preferably at 12.04% and *Streptococcus thermophilus* is present at 11.4%-12.5%, preferably at 12.04% of the total colony-forming units of the probiotic mixture.

5. Probiotic mixture according to any one of claims 1 to 4, optionally in combination with inulin, for use as a medicament.

6. Probiotic mixture for use according to claim 5, for use in the prevention and/or treatment of intestinal diseases selected among infectious and antibiotic therapy-associated diarrheas, preferably wherein the infectious or antibiotic therapy associated diarrheas are caused by *Clostridium difficile* or intestinal dysbiosis.

7. Composition comprising as sole probiotics the bacteria of the mixture as defined in any one of claims 1 to 4, optionally inulin, and at least one suitable excipient and/or additive.

8. Composition according to claim for oral use.

9. Composition according to claim 8 in a solid, semisolid, liquid or semiliquid form.

10. Composition according to claim 9 in the form of a tablet, hard or soft capsule, pill, gelatine, lozenge, powder, granules, sachet, film, drops, suspension, emulsion, solution, syrup or elixir.

11. Composition according to claim 10 in the form of a gastro-resistant tablet or capsule, or delayed-release tablet or capsule.

12. Composition according to any one of claims 7 to 11 for use as a medicament.

13. Composition for use according to claim 12, for use in the prevention and/or treatment of intestinal diseases selected among infectious and antibiotic therapy-associated diarrheas, preferably wherein the infectious or antibiotic therapy-associated diarrheas are caused by *Clostridium difficile,* and or intestinal dysbiosis.

14. Food product selected among a food supplement, beverage, nutraceutical, aliment and functional or medicated aliment comprising the mixture according to any one of claims 1 to 4, optionally inulin and at least one suitable excipient and/or additive, wherein said food product is in the form of a yogurt, juice, smoothie, extract, extracted juice, food bar or gelatine.

**Patentansprüche**

1. Probiotische Mischung bestehend aus *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* und *Streptococcus thermophilus.*

2. Probiotische Mischung nach Anspruch 1, wobei fakultativ aerob-anaerobe Bakterien (*Lactobacillus casei, Lactobacillus gasseri, Lactobacillus rhamnosus, Enterococcus faecium* und *Streptococcus thermophilus)* zu 40 ± 5 % und anaerobe Bakterien (*Bifidobacterium bifidum, Bifidobacterium breve, Bifidobacterium longum* und *Bacillus subtilis)* zu 60 ± 5 % der gesamten koloniebildenden Einheiten pro Gramm (CFU x g) der probiotischen Mischung vorhanden sind.

3. Probiotische Mischung nach Anspruch 1 oder 2, wobei Probiotika der Gattung *Bifidobacterium* zu 50 % ± 5 %, Probiotika der Gattung *Lactobacillus* zu 16,5 % ± 5 %, *Enterococcus faecium* und *Streptococcus thermophilus* insgesamt zu 23,5 % ± 5 % und *Bacillus subtilis* zu 10 % ± 5 % der gesamten koloniebildenden Einheiten pro Gramm (CFU x g) der probiotischen Mischung vorhanden sind.

4. Probiotische Mischung nach einem der Ansprüche 1 bis 3, wobei *Bifidobacterium bifidum* zu 12 %-14 %, vorzugsweise zu 13,03 % vorhanden ist; *Bifidobacterium breve* zu 11,6 %-13,1 %, vorzugsweise zu 12,3 % vorhanden ist; *Bifidobacterium longum* zu 22,8 %-25,5 %, vorzugsweise zu 24,19 % vorhanden ist; *Bacillus subtilis* zu 9,6 %-10,7 %, vorzugsweise zu 10,1 % vorhanden ist; *Lactobacillus casei* zu 5,2 %-6,9 %, vorzugsweise zu 6,1 % vorhanden ist; *Lactobacillus gasseri* zu 3,9 %-4,2 %, vorzugsweise zu 4,1 % vorhanden ist; *Lactobacillus rhamnosus* zu 5,2 %-6,9 %, vorzugsweise zu 6,1 % vorhanden ist; *Enterococcus faecium* zu 11,4 %-12,5 %, vorzugsweise zu 12,04 % vorhanden ist und *Streptococcus thermophilus* zu 11,4 %-12,5 %, vorzugsweise zu 12,04 % der gesamten koloniebildenden Einheiten der probiotischen Mischung vorhanden ist.

5. Probiotische Mischung nach einem der Ansprüche 1 bis 4, optional in Kombination mit Inulin, zur Verwendung als Arzneimittel.

6. Probiotische Mischung zur Verwendung nach Anspruch 5 zur Verwendung bei der Vorbeugung und/oder Behandlung von Darmerkrankungen, ausgewählt aus infektiösen und mit einer Antibiotikatherapie verbundenen Durchfällen, vorzugsweise wobei die infektiösen oder mit einer Antibiotikatherapie verbundenen Durchfälle durch *Clostridium difficile* oder eine Darmdysbiose verursacht werden.

7. Zusammensetzung, umfassend als einzige Probiotika die Bakterien der Mischung nach einem der Ansprüche 1 bis 4, optional Inulin und mindestens einen geeigneten Träger und/oder Zusatzstoff.

8. Zusammensetzung nach Anspruch 7 zur oralen Anwendung.

9. Zusammensetzung nach Anspruch 8 in fester, halbfester, flüssiger oder halbflüssiger Form.

10. Zusammensetzung nach Anspruch 9 in Form einer Tablette, Hart- oder Weichkapsel, Pille, Gelatine, Lutschtablette, eines Pulvers, Granulats, Sachets, Films, Tropfens, einer Suspension, Emulsion, Lösung, eines Sirups oder Elixiers.

11. Zusammensetzung nach Anspruch 10 in Form einer magensaftresistenten Tablette oder Kapsel oder einer Tablette oder Kapsel mit verzögerter Wirkstofffreisetzung.

12. Zusammensetzung nach einem der Ansprüche 7 bis 11 zur Verwendung als Arzneimittel.

13. Zusammensetzung zur Verwendung nach Anspruch 12 zur Verwendung bei der Vorbeugung und/oder Behandlung von Darmerkrankungen, ausgewählt aus infektiösen und mit einer Antibiotikatherapie verbundenen Durchfällen, vorzugsweise wenn die infektiösen oder mit einer Antibiotikatherapie verbundenen Durchfälle durch *Clostridium difficile* und/oder eine Darmdysbiose verursacht werden.

14. Nahrungsmittelprodukt, ausgewählt aus Nahrungsergänzungsmitteln, Getränken, Nutraceutika, Nahrungsmitteln und funktionellen oder medizinischen Nahrungsmitteln, umfassend die Mischung nach einem der Ansprüche 1 bis 4, optional Inulin und mindestens einen geeigneten Träger und/oder Zusatzstoff, wobei das Nahrungsmittelprodukt in Form eines Joghurts, Safts, Smoothies, Extrakts, extrahierten Safts, Nahrungsriegels oder Gelatine vorliegt.

**Revendications**

1. Mélange probiotique composé de *Bifidobacterium bifidum, Bifidobacterium breve, Bifidobacterium longum, Bacillus subtilis, Lactobacillus casei, Lactobacillus gasseri, Lactobacillus rhamnosus, Enterococcus faecium* et *Streptococcus thermophilus.*

2. Mélange probiotique selon la revendication 1, dans lequel les bactéries aérobies-anaérobies facultatives *(Lactobacillus casei, Lactobacillus gasseri, Lactobacillus rhamnosus, Enterococcus faecium* et *Streptococcus thermophilus)* sont présentes à hauteur de 40 ± 5 % et les bactéries anaérobies *(Bifidobacterium bifidum, Bifidobacterium breve, Bifidobacterium longum* et *Bacillus subtilis)* sont présentes à hauteur de 60 ± 5 % du nombre total d'unités formant colonies par gramme (UFC x g) du mélange probiotique.

3. Mélange probiotique selon la revendication 1 ou 2, dans lequel les probiotiques du genre *Bifidobacterium* sont présents à hauteur de 50 % ± 5 %, les probiotiques du genre *Lactobacillus* sont présents à hauteur de 16,5 % ± 5 %, *Enterococcus faecium* et *Streptococcus thermophilus* sont globalement présentes à hauteur de 23,5 % ± 5 % et *Bacillus subtilis* est présente à hauteur de 10 % ± 5 % du nombre total des unités formant colonies par gramme (UFC x g) du mélange probiotique.

4. Mélange probiotique selon l'une quelconque des revendications 1 à 3, dans lequel *Bifidobacterium bifidum* est présente à hauteur de 12 % à 14 %, de préférence à hauteur de 13,03 % ; *Bifidobacterium breve* est présente à hauteur de 11,6 % à 13,1 %, de préférence à hauteur de 12,3 % ; *Bifidobacterium longum* est présente à hauteur de 22,8 % à 25,5 %, de préférence à hauteur de 24,19 % ; *Bacillus subtilis* est présente à hauteur de 9,6 % à 10,7 %, de préférence à 10,1 % ; *Lactobacillus casei* est présente à hauteur de 5,2 % à 6,9 %, de préférence à hauteur de 6,1 % ; *Lactobacillus gasseri* est présente à hauteur de 3,9 % à 4,2 %, de préférence à hauteur de 4,1 % ; *Lactobacillus rhamnosus* est présente à hauteur de 5,2 %-6,9 %, de préférence à hauteur de 6,1 % ; *Enterococcus faecium* est présente à hauteur de 11,4 % à 12,5 %, de préférence à hauteur de 12,04 % et *Streptococcus thermophilus* est présente à hauteur de 11,4 % à 12,5 %, de préférence à hauteur de 12,04 % du nombre total d'unités formant colonies du mélange probiotique.

5. Mélange probiotique selon l'une quelconque des revendications 1 à 4, éventuellement en combinaison avec de l'inuline, destiné à être utilisé comme médicament.

6. Mélange probiotique destiné à être utilisé selon la revendication 5, destiné à être utilisé dans la prévention et/ou le traitement de maladies intestinales choisies parmi les diarrhées d'origines infectieuses et les diarrhées associées à l'antibiothérapie, de préférence dans lequel les diarrhées d'origines infectieuses ou associées à l'antibiothérapie sont provoquées par *Clostridium difficile* ou par une dysbiose intestinale.

7. Composition comprenant comme seuls probiotiques les bactéries du mélange tel que défini dans l'une quelconque des revendications 1 à 4, éventuellement de l'inuline, et au moins un excipient et/ou additif appropriés.

8. Composition selon la revendication 7 pour usage par voie orale.

9. Composition selon la revendication 8 sous forme solide, semi-solide, liquide ou semi-liquide.

10. Composition selon la revendication 9, sous forme de comprimé, gélule dure ou capsule molle, pilule, gélatine, pastille, poudre, granulés, sachet, film, gouttes, suspension, émulsion, solution, sirop ou élixir.

11. Composition selon la revendication 10, sous forme de gélule, capsule ou comprimé gastro-résistant, ou de gélule, capsule ou comprimé à action retardée.

12. Composition selon l'une quelconque des revendications 7 à 11 destinée à être utilisée comme médicament.

13. Composition destinée à être utilisée selon la revendication 12, destinée à être utilisée dans la prévention et/ou le traitement de maladies intestinales choisies parmi les diarrhées d'origines infectieuses et les diarrhées associées à l'antibiothérapie, de préférence dans lequel les diarrhées d'origines infectieuses ou associées à l'antibiothérapie sont provoquées par *Clostridium difficile* ou par une dysbiose intestinale.

14. Produit alimentaire choisi parmi un complément alimentaire, boisson, nutraceutique, aliment et aliment fonctionnel ou médicamenteux comprenant le mélange selon l'une quelconque des revendications 1 à 4, éventuellement de l'inuline et au moins un excipient et/ou un additif appropriés, dans lequel ledit produit alimentaire est sous forme de yaourt, jus, boisson fouettée, extrait, jus extrait, barre alimentaire ou gélatine.

phyla present in donors and recipients before and after intestinal
microbiota transplant - relative abundances

FIG. 1

FIG. 2

FIG. 3

# EP 4 009 995 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **SURAWICZ et al.** *Am J Gastroenterol,* 2013
- **DEBAST et al.** *Clin Infect Dis,* 2014
- **CAMMAROTA G. ; MASUCCI L. et al.** Randomised clinical trial: faecal microbiota transplantation by colonoscopy vs. vancomycin for the treatment of recurrent Clostridium difficile infection. *Aliment Pharmacol Ther.,* May 2015, vol. 41 (9), 835-43
- **LI M et al.** Fecal microbiota transplantation and bacterial consortium transplantation have comparable effects on the re-establishment of mucosal barrier function in mice with intestinal dysbiosis. *Front Microbiol.,* 07 July 2015, vol. 6, 692
- **PETROF et al.** Stool substitute transplant therapy for the eradication of Clostridium difficile infection. *Microbiome.,* vol. 1 (1), 3
- **LAU CS ; CHAMBERLAIN RS.** Probiotics are effective at preventing Clostridium difficile-associated diarrhea: a systematic review and meta-analysis. *Int J Gen Med.,* 22 February 2016, vol. 9, 27-37
- **SPINLER JK ; ROSS CL ; SAVIDGE TC.** Probiotics as adjunctive therapy for preventing Clostridium difficile infection - What are we waiting for?. *Anaerobe.,* October 2016, vol. 41, 51-57
- **BAKKEN JS.** Staggered and tapered antibiotic withdrawal with administration of kefir for recurrent Clostridium difficile infection. *Clin Infect Dis.,* 15 September 2014, vol. 59 (6), 858-61
- **PETROF et al.** Microbial ecosystem therapeutics: a new paradigm in medicine?. *BENEFICIAL MICROBES,* 01 March 2013, vol. 4 (1), 53-65